# EUROPEAN PATENT SPECIFICATION

(11) **EP 1 766 070 B1**
(45) Date of publication and mention of the grant of the patent: **17.12.2008**
(21) Application number: 05757545.8
(22) Date of filing: 20.06.2005
(51) Int. Cl.: C12Q 1/68

(54) **RNA BIOASSAY**
RNA-BIOTEST
BIODOSAGE D'ARN

(30) Priority: 30.06.2004 US 584461 P; 28.01.2005 US 46301
(43) Date of publication of application: 28.03.2007
(73) Proprietor: Pfizer Products Inc., Groton, CT 06340 (US)
(72) Inventor: BREES, Dominique,Jules, Jean, Emmanuel, Groton CT 06340 (US); LOY, James, Kenneth Pfizer Global R & D, Groton CT 06340 (US)
(74) Representative: England, Peter Michael
(86) International application number: PCT/IB2005/002001
(87) International publication number: WO 2006/003493

(56) References cited:
- WO-A-02/18652
- DING RUCHUANG ET AL: "Noninvasive diagnosis of BK virus nephritis by measurement of messenger RNA for BK virus VP1 in urine" TRANSPLANTATION (BALTIMORE), vol. 74, no. 7, 15 October 2002 (2002-10-15), pages 987-994, XP009053814 ISSN: 0041-1337
- KUBO K ET AL: "Detection of WT1 mRNA in urine from patients with kidney diseases" EUROPEAN JOURNAL OF CLINICAL INVESTIGATION, vol. 29, no. 10, October 1999 (1999-10), pages 824-826, XP002344649 ISSN: 0014-2972
- TATAPUDI RAVI RAJU ET AL: "Noninvasive detection of renal allograft inflammation by measurements of mRNA for IP-10 and CXCR3 in urine." KIDNEY INTERNATIONAL. JUN 2004, vol. 65, no. 6, June 2004 (2004-06), pages 2390-2397, XP002344650 ISSN: 0085-2538
- MARENGO SUSAN RUTH ET AL: "Decreased renal expression of the putative calcium oxalate inhibitor Tamm-Horsfall protein in the ethylene glycol rat model of calcium oxalate urolithiasis" JOURNAL OF UROLOGY, vol. 167, no. 5, May 2002 (2002-05), pages 2192-2197, XP002344651 ISSN: 0022-5347
- ICHIMURA T ET AL: "Kidney injury molecule-1 (KIM-1), a putative epithelial cell adhesion molecule containing a novel immunoglobin domain, is up-regulated in renal cells after injury" JOURNAL OF BIOLOGICAL CHEMISTRY, AMERICAN SOCIETY OF BIOLOGICAL CHEMISTS, BALTIMORE, MD, US, vol. 273, no. 7, 13 February 1998 (1998-02-13), pages 4135-4142, XP002079926 ISSN: 0021-9258
- TZORTZAKI ELENI G ET AL: "Gender- and age-dependent changes in kidney androgen protein mRNA expression in a knockout mouse model for nephrolithiasis." THE JOURNAL OF HISTOCHEMISTRY AND CYTOCHEMISTRY : OFFICIAL JOURNAL OF THE HISTOCHEMISTRY SOCIETY. DEC 2002, vol. 50, no. 12, December 2002 (2002-12), pages 1663-1669, XP002344718 ISSN: 0022-1554
- THULESEN J ET AL: "Renal content and output of epidermal growth factor in long-term adrenergic agonist-treated rats." REGULATORY PEPTIDES. 30 JUN 2000, vol. 90, no. 1-3, 30 June 2000 (2000-06-30), pages 69-76, XP002344719 ISSN: 0167-0115

## Description

### FIELD OF THE INVENTION

The present invention relates to methods for characterizing the pathological state of the kidney, methods of characterizing an agent, methods of evaluating the kidney protective and therapeutic characteristics of an agent and methods of monitoring the effect of a pharmaceutical agent on the kidney of a subject, related to measuring a kidney RNA marker in the urine of a subject.

### BACKGROUND OF THE INVENTION

In the pharmaceutical field, great efforts are being made to minimize the toxicological potential of pharmaceutical agents. Among the risks associated with exposure to toxic agents is the possibility of causing injury or death to cells of the body, which can result in damage to vital organs.

Mandel and Metais (1948) reported the discovery of extracellular nucleic acids in human plasma.

Lo, K.W. et al. (1999) and Kopreski, M. et al. (1999) have both reported the detection of tumor derived RNA in the plasma of cancer patients.

Chen, X.Q. et al. (2000) have proposed the use of telomerase RNA as a detection marker in the serum of breast cancer patients.

Poon, L.L. et al. (2000) have reported the presence of fetal RNA in maternal plasma.

U.S. Patent No. 6,329,179 discloses the use of tumor-derived or associated extracellular RNA found circulating in the plasma or serum fraction of blood for the detection, monitoring, or evaluation of cancer or pre-malignant conditions.

U.S. Patent No. 6,156,504 discloses the detection, identification, or monitoring of the existence, progression or clinical status of benign, pre-malignant, or malignant neoplasms in humans or animals that contain a mutation associated with the neoplasm through detection of the mutated nucleic acid of the neoplasm in plasma or serum fractions.

U.S. Patent No. 6,020,124 discloses the detection of soluble DNA for mutated genes and oncogenes in biological fluids.

As RNA is considered to be labile, researchers have examined possible mechanisms by which RNA in human plasma is protected from degradation. Hasselmann, D.O. et al. (2001) have reported that in an *in vitro* model, mRNA within apoptotic bodies released by melanoma cells was protected from degradation when incubated in human serum.

Ng, E.K.O. et al. (2002) have reported that a substantial portion of plasma mRNA is particle-associated.

Commonly assigned U.S. Patent Application No. 10/745823 discloses methods for evaluating the cell damaging potential of an agent by determining the ability of the agent to increase messenger RNA release in cells.

Conventional methods available for determining whether an agent causes tissue and cell damage in the kidney include measuring physical and chemical characteristics of urine (e.g., color, transparency, odor, volume, osmolality, specific gravity, pH and presence of sediments, protein, glucose, bilirubin, blood, urobilinogen, nitrite and enzymes), performing a renal function test, measuring blood urea nitrogen and measuring serum creatine. However, the ability to use such indicators for early detection depends upon a number of factors, including the rapidity of enzyme release and the sensitivity of detection. Hence, there exists a growing interest to uncover additional early biological indicators or biomarkers of toxicity that have higher specificity and sensitivity as compared to traditional methods for detecting toxicity.

The kidney-specific androgen-regulated protein (KAP) is expressed in the epithelial cells of the renal proximal convoluted tubules. Toole et al. (1979); Meseguer and Catterall (1987). Kidney injury molecule-1 (KIM-1), a membrane protein expressed in proximal tubule epithelial cells, is upregulated following ischemic injury as well as following treatment with the nephrotoxicants, S-(1,1,2,2-tetrafluoroethyl)-L-cysteine (TFEC), folic acid and cisplatin. Ichimura et al. (1998): Ichimura et al. (2004). Heparin-binding epidermal growth factor (HB-EGF) expression is induced in the kidney following ischemic injury and following treatment with the nephrotoxicant, mercuric chloride. Homma et al. (1995). Fibroblast growth factor 1 (FGF-1) and keratinocyte growth factor (FGF-7), members of the fibroblast growth factor family of proteins, and the FGF-7 receptor, FGFR2 IIIb, are induced in the kidney following treatment with TFEC. Ichimura et al. (1995): Ichimura et al. (1996). The water channel proteins, aquaporin 1, 2 and 3, are highly expressed in the kidney. Agre et al. (1993); Fushimi et al. (1993); and Ishibashi et al. (1994). Tamm-Horsfall glycoprotein is expressed in kidney epithelial cells and localizes to the thick ascending limbs of the loops of Henle and the distal convoluted tubules of the kidney. Sikri et al. (1981); and Zhu, X. et al. (2002). Expression of the early growth response gene, Egr-1, the proto-oncogene, c-fos, and the stress response gene, hsp 70, is activated following ischemic injury of the kidney. Ouellette et al. (1990); Bardella and Comolli (1994).

### SUMMARY OF THE INVENTION

The present invention relates, in part, to methods of characterizing the pathological state of the kidney of a subject comprising, measuring the amount of a kidney RNA marker in a urine sample obtained from a mammalian subject, preferably a human subject wherein said kidney RNA marker is kidney-specific androgen-regulated protein (KAP). In a preferred embodiment, said method further comprises characterizing said subject as having a pathological state of the kidney, provided said urine sample contains at least a two fold higher amount of said kidney RNA marker than that which is measured in a control subject having no pathological state of the kidney.

An additional aspect of this invention provides methods of characterizing an Agent comprising, treating a non-human mammalian subject with an Agent, and measuring the effect of said Agent on the amount of an kidney RNA marker released into the urine of said subject; wherein said kidney RNA marker is KAP.

A further aspect of this invention relates to methods for evaluating the kidney protective characteristics of an Agent comprising, treating a non-human mammalian subject with a first Agent wherein said first Agent is characterized by increasing the amount of a kidney RNA marker released into the urine of said subject, treating said subject with a second Agent, and measuring the effect of said second Agent In reducing the amount of said kidney RNA marker released into the urine of said subject caused by said first Agent; wherein said kidney RNA marker KAP.

Another aspect of this invention provides methods of monitoring the effect of a pharmaceutical Agent on the kidney of a human subject, treated with a pharmaceutical Agent, and measuring the effect of said Agent on the amount of a kidney RNA marker released into the urine of said subject; wherein said kidney RNA marker KAP.

### Definitions

The terms used herein have their usual meaning in the art. However, to even further clarify the present invention and for convenience, the meaning of certain terms and phrases employed in the specification, including the examples and appended claims are provided below.

"Agent" means a chemical, biological or physical means for producing an effect. An Agent may be one or a combination of two or more chemical or biochemical substances, biological pathogens or physical perturbations.

"Nucleotide sequence" and "polynucleotide" mean DNA or RNA, whether in single-stranded or double-stranded form. The term "complimentary nucleotide sequence" means a nucleotide sequence that anneals (binds) to a another nucleotide sequence according to the pairing of a guanidine nucleotide (G) with a cytidine nucleotide (C) and adenosine nucleotide (A) with thymidine nucleotide (T), except in RNA where a T is replaced with a uridine nucleotide (U) so that U binds with A.

"Pathological state" means a state of biological abnormality and includes abnormal states of an organ, tissue type or cell type. The term includes abnormality regardless of the cause, and may include diseases, arising, for example, from physiological abnormalities, genetic abnormalities or pathogenic agents, physical injuries, or toxicities caused by xenobiotic agents.

"RNA marker" means an RNA polynucleotide, or a fragment thereof, having a specific sequence that is transcribed from the DNA genetic information of a cell. The term RNA marker includes mRNA polynucleotides, or fragments thereof, that have been processed by a cell, for example, by 5' capping, RNA splicing and 3' polyadenylation, following copying from the corresponding DNA sequence. When "RNA marker" is preceded by the name of an organ, tissue type or cell type (e.g., "kidney RNA marker"), the term means that the RNA marker is transcribed in that organ, tissue type or cell type. For the purposes of this invention, such use of the term would preferably refer to RNA markers which are transcribed only in the designated organ, tissue type or cell type. However, the term also includes an RNA marker that can be used to identify the particular organ, tissue type or cell type of interest within the context of the invention. For example, in the methods of this invention that involve characterizing a pathological state of the kidney by measuring a kidney RNA marker in urine, the particular RNA marker does not have to be one exclusively expressed in the kidney, provided any expression, for example, in a second organ or tissue, is unlikely to be detected in urine as a result of pathology of that second organ or tissue.

### Abbreviations:

The abbreviations used herein have their usual meaning in the art. However, to even further clarify the present invention, for convenience, the meaning of certain abbreviations are provided as follows: "°C" means degrees Centigrade; "cDNA" means complementary DNA; "CT' means number of amplification cycles; "dL" means deciliter(s), "DNA" means deoxyribonucleic acid; "EDTA" means ethylenediamine tetra-acetic acid; "g" means gram: "kg" means kilogram; "NaOH" means socium hydroxide; "mRNA" means messenger RNA: "mg" means milligram; "mL" means milliliter; "ng" means nanogram; "PCR" means polymerase chain reaction: "PBS" means phosphate buffered saline; "RNA" means ribonucleic acid; "RPM" means revolutions per minute; and "RT-PCR" means reverse transcriptase polymerase chain reaction.

### BRIEF DESCRIPTION OF THE DRAWINGS

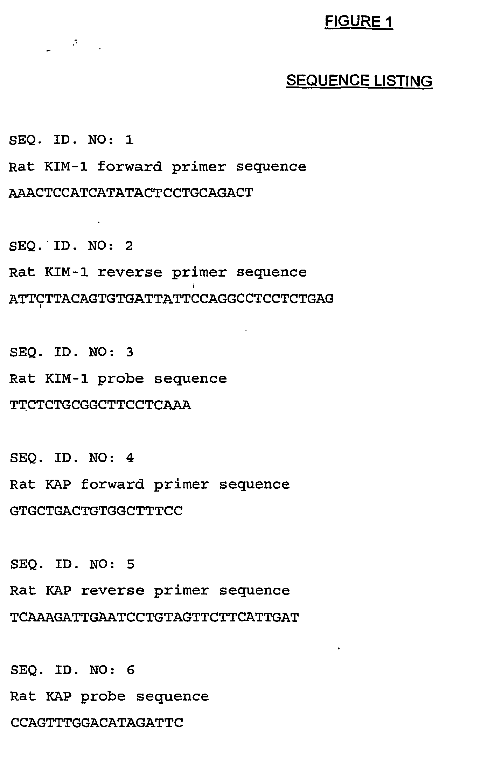

Figure 1 shows the sequences of PCR primers and PCR probes that may be used to amplify and detect rat KIM-1 and KAP.

### DETAILED DESCRIPTION OF THE INVENTION

During normal cell turnover, mammalian cells release RNA that is detectable in the plasma. This invention is based, in part, on the discovery that the release of RNA by cells of the kidney is exacerbated by cellular injury caused by chemical agents, that this effect is detectable in the urine of a mammal and is a useful indicator of kidney injury. One aspect of this invention makes use of the tissue-type specific and cell-type specific nature of gene transcription (*i.e*., for a particular tissue or cell type, that some genes may be transcribed and others may not) and the tissue-type and cell-type specific splicing arrangement of certain gene transcripts. RNAs that are distinctive of a particular tissue-type or cell-type may be used as biomarkers of biological, physiological or toxicological injury for specific organs, tissues or cell types.

The invention provides methods of testing potentially toxic Agents, Identification of therapeutic Agents, testing the efficacy of therapeutic Agents, and characterizing the pathologic state of the kidney based upon the release of RNA by kidney cells into the urine as an indicator of cell injury or cell death. RNA detection techniques known to those skilled in the art or that will be apparent based upon this disclosure are useful in the practice of this invention.

One embodiment provides *in vivo* non-invasive methods for early detection of the nephrotoxicity of an Agent. According to such methods, the urine of a subject is tested to determine the level of RNA of a specific kidney RNA marker, i.e. KAP (as described, for example, in Toole et al. (1979) and Meseguer and Catterall (1987)). The toxicity of the Agent is evaluated based upon its ability to cause the cellular release of RNA as measured in the urine of the test subject. Such methods would be useful as *in vivo* assays for the evaluation of the nephrotoxicity of test Agents, using, for example, test animals. The methods may also be used clinically to non-invasively monitor patients following drug treatment as an early warning of possible nephrotoxicity.

In a further embodiment, the present invention provides non-invasive methods for evaluating the kidney protective or therapeutic characteristics of a test Agent. According to such methods, a reduction in the amount of a kidney RNA marker (specifically KAP) released, for example, by cells of the kidney that are treated with an Agent that is known to increase the release of KAP will indicate that the test Agent has a desired protective or therapeutic effect on the kidney.

In the practice of the methods of this invention, urine is collected from a subject into a sterile collection tube or vial. Preferably, all collection equipment and containers are free of any extraneous RNA. The urine is then preferably flash frozen and stored at 80 °C.

Generally, the detection of RNA for the practice of the methods of this invention entails a multistep process: (1) extraction of RNA; (2) amplification, which may involve reverse transcription of RNA to its cDNA; and (3) detection. While not intended as a commitment to any particular theory or mechanism, it is believed that extracellular RNA is present extracellularly as protein-RNA, lipoprotein-RNA complexes, lipid-RNA or DNA-RNA complex, and that such complexing may interfere with amplification and detection of RNA levels. Hence, it is preferable according to this theory to use an extraction step in order to dissociate the RNA from its associated complexes, prior to amplification and detection.

Any of a number of nucleic acid extraction methods known to those with skill in the art may be used in the practice of the invention. Although many of the published methods are intended for extraction of intracellular RNA, they may be used as described in the literature or with modification that will be apparent to those with skill in the art, based on the present disclosure. For example, extracellular RNA may be extracted using silica particles, glass beads, or diatoms, as in methods described in Boom et al. (1990) or Cheung et al. (1994).

In an exemplary method for extracting RNA from cell growth media for the *in vitro* methods of the invention, the media that has been separated from the cells by methods well known in the art (for example, by centrifugation or filtration) are treated with an RNA stabilizing agents, such as a chaotropic substance, for example, guanidimium (iso)thiocyanate as described in U.S. Patent No. 5,234,809. The RNA is then bound to a solid binding agent, such as silica particles. The RNA-solid phase may then be separated from the liquid phase by filtration. The RNA may then be eluted from the silica particles using the RNA may be eluted using a buffer consisting of 10 mM Tris-HCl, one mM EDTA (pH 8.0).

In an exemplary extraction method from urine, about 0.1 mL to about 1.0 mL of urine are mixed with 40 microliters of an aqueous silica suspension, prepared as described below, and about 900 microliters of lysis buffer, prepared as described as described below, and mixed at room temperature for about 10 minutes. The mixture is then centrifuged at 12,000x g for one minute and the supernatant is removed. The resulting silica-RNA pellet is then washed twice with about 450 microliters of a washing buffer, prepared as described below, followed by about one ml of 70% (vol/vol) ethanol. Finally, the pellet is washed with one mL acetone and dried at about degrees 56°C for about 10 minutes. RNA is then eluted from the silica pellet by suspending in about 20 to 50. microliters of diethyl procarbonate-treated water at 56°C for about 10 minutes followed by centrifugation at about 12,000 times gravity for about three minutes. Alternatively, the RNA may be eluted using a buffer consisting of 10 mM Tris-HCl, one mM EDTA (pH 8.0) and an RNase inhibitor (*e.g*., RNAsin®, Promega, Madison WI), with or without a proteinase, such as proteinase K, according to the method described in Boom et al. (1991). The resulting RNA containing supernatant is recovered for amplification and detection.

The aqueous silica suspension described above may be prepared by suspending 60 grams of silicon dioxide (Sigma-Aldrich Corp., St. Louis, MO) in 500 mL of demineralized sterile double-distilled water. The suspension is then allowed to settle for about 24 hours at room temperature. The supernatant is removed and the particles are resuspended in demineralized, sterile double-distilled water added to equal a volume of 500 milliliters. Once the suspension has settled, about 440 milliliters of the supernatant is removed and the the pH of the suspension is adjusted to about and pH 2 using hydrochloric acid.

The lysis buffer described above is prepared by dissolving 120 grams of guinidine thiocyanate into 100 mL of 0.1 M Tris hydrochloride (Tris-HCl) (pH 6.4), and 22 milliliters of 0.2 M EDTA, adjusted to pH 8.0 with NaOH, and 2.6 grams of Triton X-100 (Sigma-Aldrich Corp.).

The washing buffer described above is prepared by dissolving 120 grams of guinidine thiocyanate into 100 milliliters of 0.1 M Tris-HCl (pH 6.4).

Alternative methods may be used to extract RNA, including but not limited to centrifugation through a cesium chloride gradient, for example, as described by Chirgwin et al. (1979), and coprecipitation of extracellular RNA from plasma or serum with gelatin, for example, as generally described in Foumie et al. (1986). Other methods of RNA extraction will be apparent to those skilled in the art based upon the present disclosure.

Preferred methods of RNA extraction from urine include the use of an RNeasy^{™} kit (cat. no. 74124, Qiagen Inc., Valencia, CA) or a QIAamp Viral RNA kit (Qiagen Inc.).

Prior to amplification and detection, it may be desirable to further purify the RNA by removal of trace DNA. Further purification may be accomplished using DNase according to methods described in Rashtchian, A. (1994).

RNA which has been extracted, and, if desired, purified, as described above, may be amplified using a nucleic acid amplification assay for the desired kidney RNA marker, i.e. KAP.

Any nucleic acid amplification assay capable of permitting detection of small numbers of RNA molecules may be used in the practice of the invention. Applicable assays include, but are not limited to, reverse transcriptase polymerase chain reaction (RT-PCR), ligase chain reaction (*see*, *for example,* Abravaya, K. et al. (1995), branched DNA signal amplification (*see, for example,* Jrdea, M.S. et al. (1993)), isothermal nucleic acid sequence based amplification (NASBA) (*see, for example,* Kievits, T. et al. (1991)), and other self-sustained sequence replication assays.

The preferred embodiment for amplification of RNA for this invention is through the use of RT-PCR. Methods for amplification of RNA using RT-PCR are well known to those with skill in the art and are well described in the literature (see, for example, Ausubel et al. (1994) and Innis et al. (1990)).

As those skilled in the art will appreciate based upon the present disclosure, the choice of primers that are to be used for amplification and probes for detection of the amplification product, for example by RT-PCR, will depend upon the types of cells that are evaluated through the methods of the invention. For the *in vitro* methods of this invention, such evaluation involves the use of cell cultures comprising one or more cell types.

Accordingly, sets of probes and primers should target one or more transcribed genes of such cells such that the transcription thereof may be used to identify those cells. Preferably, such probes and primers will target RNAs that are unique to the particular cells-type. It is also preferable that such genes be transcribed at a relatively high level, and preferably continuously, regardless of the state of the cell or conditions affecting the cell.

Methods for preparing and using probes and primers are well known in the art, and are described, for example, in Sambrook et al. (1989), Ausubel et al. (1994) and Innis et al. (1990). PCR primer pairs can be derived from known sequences, for example, by using computer programs intended for that purpose such as Primer (Version 0.5, 1991, Whitehead Institute for Biomedical Research, Cambridge MA).

Oligonucleotides for use as primers are selected using software known in the art for such purpose. For example, OLIGO^{®} version 6 software (Molecular Biology Insights, Inc., Cascade, CO, www.oligo.net) is useful for the selection of PCR primer pairs of up to 100 nucleotides each, and for the analysis of oligonucleotides and larger polynucleotides of up to 5,000 nucleotides from an input polynucleotide sequence of up to 32 kilobases. Similar primer selection programs have incorporated additional features for expanded capabilities. For example, the PrimOU primer selection program (available to the public from the Genome Center at University of Texas, South West Medical Center, Dallas TX, ftp://ftp.genome.ou.edu/pub/programs/primou src.tar) is capable of choosing specific primers from megabase sequences and is thus useful for designing primers on a genome-wide scope. The Primer3, version 0.9, primer selection program (available to the public from the Whitehead Institute/MIT Center for Genome Research, Cambridge MA, http://www-genome.wi.mit.edu/genome software/other/primer3.html) allows the user to input a "mispriming library," in which sequences to avoid as primer binding sites are user-specified. Primer3 is useful, in particular, for the selection of oligonucleotides for microarrays. (The source code for the latter two primer selection programs may also be obtained from their respective sources and modified to meet the user's specific needs.) The PrimeGen program (available to the public from the UK Human Genome Mapping Project Resource Centre, Cambridge UK, http://www.hgmp.mrc.ac.uk/Registered/Option/primegen.html) designs primers based on multiple sequence alignments, thereby allowing selection of primers that hybridize to either the most conserved or least conserved regions of aligned nucleic acid sequences. Hence, this program is useful for identification of both unique and conserved oligonucleotides and polynucleotide fragments. Other oligonucleotide selection methods will be apparent to those with skill in the art based upon the present description.

RNA that has been extracted and/or amplified, as described above, may be detected using a detection method. Methods of RNA detection are well known by those with skill in the art, and are described, for example, in Sambrook et al. (1989), Ausubel et al. (1994) and Innis et al. (1990).

A preferred detection system uses real-time detection of RNA during PCR cycles using an integrated thermal cycler, a fluorescence inducing light source and detector. An exemplary instrument to perform such detection is the ABI PRISM® 7700 Sequence Detection System (Applied Biosystems, Foster City, CA).

Alternative methods for detection of RNA will be apparent to those with skill in the art based upon the present description. Such methods may include, for example, gel electrophoresis followed by Southern blot analysis (*see, for example*, Nguyen, T.D. (1989)), ELISA detection methods (*see for example,* Landgraf, A. et al. (1991), Coutlee, F. et al. (1989) and Bobo, L. et al. (1990)) and methods using electrochemiluminescence detection (*see, for example*, Blackburn, G.F. (1991) and DiCesare, J. et al. (1993)). Electrophoresis detection methods may involve a comparison of bands of two or more RNA populations. Bands present on an autoradiograph of one gel from one RNA population, and not present on another, correspond to the presence of a particular RNA in one population and not in the other, and thus indicate a gene that is likely to be differentially expressed (*see, for example,* Williams, J.G. (1990), Welsh, J. et al. (1990), Woodward, S.R. (1992), Nadeau, J. H. (1992), Liang, P. et al. (1992), Welsh, J. et al. (1992) and Liang, P. et al. (1993)).

Finally, detection methods for the present invention may involve the use of arrays or microarrays. Arrays and microarrays are sets of distinct polynucleotides or oligonucleotides synthesized on a substrate, such as paper, nylon or other type of membrane, filter, chip, glass slide, or any other suitable solid support. In a preferred embodiment, arrays and microarrays may be prepared and used according to the methods described in U.S. Patent No. 5,837,832, PCT Patent Application Publication Number W0 95/11995, Lockhart et al. (1996) and Schena, M. *et* al. (1996). In other embodiments, such arrays are produced by the methods described in U.S. Patent No. 5,807,522.

It is believed that one skilled in the art can, using the present description, including the examples, drawings, sequence listings and appendant claims, utilize the present invention to its fullest extent. The following Examples are to be construed as merely illustrative of the practice of the invention and not limitative of the remainder of the disclosure in any manner whatsoever.

### EXAMPLES

### Example 1

On days 0 and 1, voided urine from four male Sprague-Dawley rats (Charles River Laborator es, Wilmington, MA) was collected, once in the morning and once in the evening. Voiding was accomplished by manually stimulating the abdomen and thorax of each rat while holding it over RNA free aluminum oil. Urine from all rats for each time period was pooled, flash frozen and stored at 80 °C. At the beginning of day 2, each rat was administered cisplatin by intraperitoneal injection at a dose of 10 mg/kg. On days 2 and 3, voided urine was again collected, once in the morning and once in the evening, pooled and flash frozen. On day 4, voided urine was collected in the morning, pooled and flash frozen. The rats were then euthanized and kidneys were collected by necropsy. Kidney tissue was fixed in 10% neutral buffered formalin and processed for histopathology by trimming, dehydrating, embedding in paraffin, sectioning, mounting on glass slides and staining with hematoxylin and eosin stains. All animals exhibited mild necrosis of the tubular epithelial cells characterized by individual cell necrosis associated with areas of tubular epithelial cell regeneration.

A portion of urine for each time period (650 µl) was used for RNA extraction using the RNeasy^{™} mini kit (Qiagen Inc., Valencia, CA) according to manufacturer instructions. The urine was analyzed using RT-PCR for presence of the RNA markers, KIM-1 and KAP.

For KIM-1, 5'-AAACTCCATCATATACTCCTGCAGACT-3' was used as the forward primer, 5'-ATTCTTACAGTGTGATTATTCCAGGCCTCCTCTGAG-3' as the reverse primer and 5'-TTCTCTGCGGCTTCCTCAAA-3' as the probe.

For KAP, 5'-GTGCTGACTGTGGCTTTCC-3' was used at the forward primer, 5'-TCAAAGATTGAATCCTGTAGTTCTTCATTGAT-3' as the reverse primer and 5'-CCAGTTTGGACATAGATTC-3' as the probe. RT-PCR was performed on a ABI PRISM® 7700 Sequence Detection System (Applied Biosystem).

The results of Example 1 are provided in Tables 1 and 2 below.

**Table 1. KIM-1**

| Day | 0 | 1 | 2 | 3 | 4 |
|---|---|---|---|---|---|
| | pre-dosing | pre-dosing | post-dosing | post-dosing | post-dosing |
| Mean CT | 39.35 | 39.14 | 39.88 | 37.39 | 36.31 |
| SD | 1.28 | 1.51 | 0.22 | 0.92 | 1.46 |
| Fold change* | | | 0 | 3.6 | 7.6 |

**Table 2. KAP**

| Day | 0 | 1 | 2 | 3 | 4 |
|---|---|---|---|---|---|
| | pre-dosing | pre-dosing | post-dosing | post-dosing | post-dosing |
| Mean CT | 30.41 | 30.55 | 29.97 | 28.23 | 26.93 |
| SD | 1.7 | 1.36 | 2.16 | 1.22 | 1.77 |
| Fold change* | | | 1.4 | 4.8 | 11.7 |

| | | | | | |
|---|---|---|---|---|---|
| * Fold change is calculated as 2^{N} wherein N equals the mean CT pre-dosing minus the mean CT of the day of dosing. | | | | | |

Example 1 illustrates the methods of this invention related to measuring a kidney RNA marker in the urine of a subject.

### References

Abravaya, K. et al. (1995), Nucleic Acids Research, 23, 675-682;
Agre, P. et al. (1993), Am. J. Physiol., 265, F463-F476;
Ausubel et al. (1994), Current Protocols in Molecular Biology, John Wiley & Sons, Hoboken, NJ;
Bardella, L. and Comolli, R. (1994), Exp. Nephrol., 2, 58-65;
Blackburn, GF et al. (1991), Olin Chem, 37, 1534-1539;
Bobo, L. et al. (1990), J. din Micra, 28,1968-1973;
Boom, R. et al. (1990), J. Clin. Microbiology, 28, 495-503;
Boom, R. et al. (1991), J. Clin. Microbiology, 29, 180-1811;
Bonfacino et al. (1998), Current Protocols in Cell Biology, John Wiley & Sons, Inc., Hoboken, NJ;
Chen, XQ et al. (2000), Clin. Cancer Res., 6, 3823-3826;
Cheung et al. (1994), J Clin. Microbiology, 32, 2593-2597;
Chirgwin, J.M. et al. (1979), Biochemistry, 18, 5294-5299;
Coutlee, F. et al. (1989), Analytical Biochemistry, 181, 96-105;
DiCesare, J. et al. (1993), BioTechniques, 15, 152-157;
Fournie, GJ et al. (1986), Analytical Biochemistry, 158, 250-256;
Diehn, M. et al. (1993), Nat. Genet., 25, 58-62;
Duncan, JR et al. (1994), Veterinary Laboratory Medicine, Clinical Pathology, Third edition. Iowa State Press, Ames, Iowa;
Freshney, R.I. (1993), Culture of Animal Cells. A Manual of Basic Techniques, 3rd ed. Wiley-Liss, New York, NY;
Fushimi, K. et al. (1993), Nature, 361, 549-552;
Hasselmann, DO (2001), Clin. Chem., 47,1488-1489;
Hayward R.E. et al. (1993), Mol. Microbiol., 35, 6-14;
Homma T. et al. (1995), J. Clin. Invest., 96, 1018-1025;
Ichimura T. et al. (1996), Am. J. Physiol. Renal Fluid Electrolyte Physiol., 269: F653-F662;
Ichimura T. et al. (1996), Am. J. Physiol. Renal Fluid Electrolyte Physiol., 271, F967-F976;
Ichimura, T. et al. (1998), J. Biol. Chem., 273, 4135-4142;
Ichimura, T. et al. (2004), Am. J. Physiol. Renal Physiol., 286, F552-563;
Innis et al. (1990), PCR Protocols, A Guide to Methods and Applications, Academic Press, San Diego CA;
Ishibashi, K. et al. (1994), Proc. Natl. Acad. Sci. USA, 91, 6269-6273;
Jrdea, MS et al. (1993), AIDS, 7 (supp. 2), S11-514;
Johannes G. et al. (1993), Proc. Natl. Acad. Sci. USA, 96, 13118-23;
Kievits, T. et al. (1991), J. Virological., Methods 35, 273-286;
Kopreski, M. et al. (1999), Clin. Cancer Res., 5, 1961-1965;
Landgraf, A. et al. (1991), Analytical Biochemistry, 198, 86-91;
Liang, P. et al., (1992), Science, 257, 967;
Liang, P. et al. (1993), Nucl. Acids Res., 3269; Lo, K.W. et al. (1999), Clin. Chem. 45, 1292-1294;
Lockhart et al. (1996), Nat. Biotech., 14, 1675-1680;
Mandel and Metais (1948), C.R. Acad. Sci. Paris, 142, 241-243;
Maines et al. (1998), Current Protocols In Toxicology, John Wiley & Sons, Inc., Hoboken, NJ;
Meseguer, A. and Catterall, JF (1987), Molecular Endocrinology, 1, 535-541;
Nadeau, JH (1992), Mamm. Genome, 3, 55;
Nguyen, TD (1989), BioTechniques, 7, 238-240;
Ng, EKO. (2002), Clin. Chem., 48, 1212-1217;
Ouellette AJ et al. (1990), J. Clin. Invest., 85, 766-771;
Poon, L.L. et al. (2000), Clin. Chem.. 46,1832-1834;
Putnam, K.P. et al. (2002), Toxicol. In Vitro, 16, 599-607;
Rashtchian, A. (1994), PCR Methods Applic., 4, S83-S91;
Sambrook et al. (1989), Molecular Cloning: A Laboratory Manual, Cold Spring Harbor Press, Plainview NY;
Schena, M. et al. (1996), Proc. Natl. Acad. Sci., 93, 10614-10619;
Scherf, U. et al. (1993), Nat. Genet. 24, 236-244;
Schmidt, DM and Ernst, JD (1995), Anal. Biochem., 232, 144-146;
Sikri, KL. et al. (1981), J. Anat., 132, 597-605;
Toole JJ, et al. (1979), Cell, 17, 441-448;
Tsui, N.Y.B. (2002), Clin. Chem., 48, 1647-1653;
Welsh, J. et al., (1990), Nucl. Acids Res., 18, 7213;
Welsh, J. et al. (1992), Nucl. Acid Res. 20, 4965;
Williams, J.G. (1990), Nucl. Acids Res., 18, 6531;
Woodward, S.R., (1992), Mamm. Genome, 3, 73;
Yukimasa, et al. (2000), Kidney International, 57, 321-331;
Zhu, X. et al. (2002), Am J Physiol Renal Physiol., 282, F608-617.

## Claims

1. A method of characterizing the pathological state of the kidney of a subject comprising measuring the amount of a kidney RNA marker in a urine sample obtained from a mammalian subject wherein said kidney RNA marker is RNA having a specific sequence that is transcribed from kidney-specific androgen-regulated protein (KAP) DNA.

2. A method of claim 1 further comprising characterizing said subject as having a pathological state of the kidney, provided said urine sample contains at least a two fold higher amount of said kidney RNA marker than that which is measured in a control subject having no pathological state of the kidney.

3. A method of claim 1 or claim 2 wherein said subject is a human.

4. A method of characterizing an agent comprising:
treating a non-human mammalian subject with an agent; and
measuring the effect of said agent on the amount of a kidney RNA marker released into the urine of said subject; wherein said kidney RNA marker is KAP.

5. A method of evaluating the kidney protective or therapeutic characteristics of an agent comprising:
treating a non-human mammalian subject with a first agent wherein said first agent is **characterized by** increasing the amount of a kidney RNA marker released into the urine of said subject;
treating said subject with a second agent; and
measuring the effect of said second agent in reducing the amount of said kidney RNA marker released into the urine of said subject caused by said first agent;
wherein said kidney RNA marker is KAP.

6. A method of monitoring the effect of a pharmaceutical agent on the kidney of a human subject:
treated with a pharmaceutical agent and comprising measuring the effect of said agent on the amount of a kidney RNA marker released into the urine of said subject; wherein said kidney RNA marker is KAP.

## Patentansprüche

1. Verfahren zum Charakterisieren des pathologischen Zustands der Niere eines Subjekts, umfassend das Messen der Menge eines RNA-Markers der Niere in einer Urinprobe, die von einem Säugersubjekt erhalten wurde, wobei der RNA-Marker der Niere RNA mit einer spezifischen Sequenz ist, die von der DNA des nierenspezifischen Androgen-regulierten Proteins ("kidney-specific androgen-regulated protein") (KAP) transkribiert ist.

2. Verfahren nach Anspruch 1, weiterhin umfassend das Charakterisieren des Subjekts, als einen pathologischen Zustand der Niere aufweisend, vorausgesetzt dass die Urinprobe mindestens eine zweifach höhere Menge des RNA-Markers der Niere enthält als diejenige, die bei einem Kontrollsubjekt ohne pathologischen Zustand der Niere gemessen wird.

3. Verfahren nach Anspruch 1 oder 2, wobei das Subjekt ein Mensch ist.

4. Verfahren zum Charakterisieren eines Mittels, umfassend:
Behandeln eines nicht menschlichen Säugersubjekts mit einem Mittel; und
Messen der Wirkung des Mittels auf die Menge an RNA-Marker der Niere, die in den Urin des Subjekts freigesetzt wird, wobei der RNA-Marker der Niere KAP ist.

5. Verfahren zum Beurteilen der nierenschützenden oder therapeutischen Eigenschaften eines Mittels, umfassend:
Behandeln eines nicht menschlichen Säugersubjekts mit einem ersten Mittel, wobei das erste Mittel **dadurch gekennzeichnet ist, dass** es die Menge eines RNA-Markers der Niere, die in den Urin des Subjekts freigesetzt wird, erhöht;
Behandeln des Subjekts mit einem zweiten Mittel; und
Messen der Wirkung des zweiten Mittels beim Verringern der Menge des RNA-Markers der Niere, die verursacht durch das erste Mittel in den Urin des Subjekts freigesetzt wird,
wobei der RNA-Marker der Niere KAP ist.

6. Verfahren zum Überwachen der Wirkung eines pharmazeutischen Mittels auf die Niere eines menschlichen Subjekts, das mit einem pharmazeutischen Mittel behandelt wird, umfassend
das Messen der Wirkung des Mittels auf die Menge eines RNA-Markers der Niere, die in den Urin des Subjekts freigesetzt wird, wobei der RNA-Marker der Niere KAP ist.

## Revendications

1. Procédé de caractérisation de l'état pathologique du rein d'un sujet, comprenant la mesure de la quantité d'un marqueur ARN du rein dans un échantillon d'urine obtenu auprès d'un sujet mammalien, ledit marqueur ARN du rein étant un ARN ayant une séquence spécifique qui est transcrite à partir de l'ADN de la protéine régulée par les androgènes spécifique du rein (KAP).

2. Procédé selon la revendication 1, comprenant en outre la caractérisation, chez ledit sujet, d'un état pathologique du rein, si ledit échantillon d'urine contient une quantité dudit marqueur ARN du rein au moins double de celle qui est mesurée chez un sujet témoin ne présentant pas d'état pathologique du rein.

3. Procédé selon la revendication 1 ou la revendication 2, dans lequel ledit sujet est un être humain.

4. Procédé de caractérisation d'un agent, comprenant :
le traitement d'un sujet mammalien non-humain avec un agent ; et
la mesure de l'effet dudit agent sur la quantité d'un marqueur ARN du rein libéré dans l'urine dudit sujet ; ledit marqueur ARN du rein étant la KAP.

5. Procédé d'évaluation des caractéristiques d'un agent en matière de protection ou de thérapie du rein, comprenant :
le traitement d'un sujet mammalien non-humain avec un premier agent, ledit premier agent étant **caractérisé par** l'augmentation de la quantité d'un marqueur ARN du rein libérée dans l'urine dudit sujet ;
le traitement dudit sujet avec un second agent ; et
la mesure de l'effet dudit second agent sur la réduction de la quantité dudit marqueur ARN du rein libéré dans l'urine dudit sujet en raison de l'action dudit premier agent ;
ledit marqueur ARN du rein étant la KAP.

6. Procédé de surveillance de l'effet d'un agent pharmaceutique sur le rein d'un sujet humain traité avec un agent pharmaceutique, le procédé comprenant la mesure de l'effet dudit agent sur la quantité d'un marqueur ARN du rein libéré dans l'urine dudit sujet ; ledit marqueur ARN du rein étant la KAP.


### SEQUENCE LISTING

<110> Pfizer Inc.
   Brees, Dominique
   Loy, James K.
<120> RNA Bioassay
<130> PC32066A
<150> US 60/584,461
   <151> 2004-06-30
<160> 6
<170> PatentIn version 3.3
<210> 1
   <211> 27
   <212> DNA
   <213> Rattus rattus
<400> 1
   aaactccatc atatactcct gcagact 27
<210> 2
   <211> 36
   <212> DNA
   <213> Rattus rattus
<400> 2
   attcttacag tgtgattatt ccaggcctcc tctgag 36
<210> 3
   <211> 20
   <212> DNA
   <213> Rattus rattus
<400> 3
   ttctctgcgg cttcctcaaa 20
<210> 4
   <211> 19
   <212> DNA
   <213> Rattus rattus
<400> 4
   gtgctgactg tggctttcc 19
<210> 5
   <211> 32
   <212> DNA
   <213> Rattus rattus
<400> 5
   tcaaagattg aatcctgtag ttcttcattg at 32
<210> 6
   <211> 19
   <212> DNA
   <213> Rattus rattus
<400> 6
   ccagtttgga catagattc 19